# EUROPEAN PATENT APPLICATION

(11) **EP 4 119 174 A1**
(43) Date of publication of application: **18.01.2023**
(21) Application number: 21305985.0
(22) Date of filing: 15.07.2021
(51) Int. Cl.: A61M 5/31, A61M 5/32

(54) **TIP COVER HAVING A CROSS-LINKED SILICONE LAYER AND METHOD OF FORMING SUCH TIP COVER**

(71) Applicant: Becton Dickinson France, 38800 Le Pont de Claix (FR)
(72) Inventor: POUGET, Gaelle, 38100 GRENOBLE (FR); BOURGEOIS, Réjane, 38000 GRENOBLE (FR)
(74) Representative: Regimbeau

(57) **Abstract**

The present disclosure relates to a method for treating a tip cover (1, 100) for an injection device (20, 120) to reduce the pull out force of the tip cover (1, 100). The method comprises applying a quantity of a silicone oil to an inner surface (10, 110) of a tip cover (1, 100), the inner surface (10, 110) configured to engage an outer surface of a tip of an injection device and treating the silicone oil to form a cross-linked silicone coating on the inner surface (10, 110) of the tip cover (10).

## Description

### TECHNICAL FIELD

The present disclosure relates generally to a tip cover, an injection device comprising the tip cover, and a method of forming the tip cover.

### BACKGROUND

Injection devices, such as syringes, are medical delivery devices configured to administer a medicinal fluid (e.g., pharmaceutical, medicament) to a patient. Syringes may be sold in a prefilled form, wherein medicinal fluid is disposed within the syringe at, for example, the time of manufacture of the syringe. Syringes typically include a container for containing the medicinal fluid having an end piece including a fluid passageway through which the medicinal fluid is expelled from the container.

In some instances, a needle may be attached to the end piece of the syringe in order to prick a patient's skin and administer the medicinal fluid to the patient. A needle cover may be mounted on the end piece so as to enclose the needle. The needle cover may comprise an inner needle shield, which may be made from an elastomeric material, and may further comprise an outer needle shield, which may be formed of a rigid material and disposed around the inner needle shield. The needle cover may prevent injury to a person handling the injection device. Further, the inner needle shield may sealingly contact the needle and/or the end piece in order to shield the needle and the end piece from any contamination of the medicinal fluid from an outside environment and/or in order to prevent leakage of the medicinal fluid from the syringe.

Alternatively, the syringe may be needle-free, and the end piece of the syringe may be provided with a fluid passageway of the end piece through which the medicinal fluid is expelled from the container when a needle is connected to said end piece. Before connecting the syringe, a tip cap may be mounted on the end piece. Like the needle cover, the tip cap may sealingly contact the end piece in order to shield the end piece from any contamination of the medicinal fluid from an outside environment and/or in order to prevent leakage of the medicinal fluid from the syringe.

In order to use the syringe, the needle cover or tip cap must be removed. In order to remove the needle cover or tip cap, the user must grip both the syringe and the needle cover or tip cap and pull the needle cover or tip cap by exerting a pulling force thereon, which force may be significant.

The force needed to remove the needle cover or tip cap is measured by a physical parameter known as "pull out force" or "POF." In order for the injection device to be usable by any user, including users having a reduced strength due to, for example, a disease, the pull out force must be sufficiently low. However, the pull out force must also be sufficiently high so as to avoid unintentional removal of the needle cover or tip cap during transport or storage for instance.

### BRIEF SUMMARY

An objective of the present disclosure is to provide a tip cover having a reduced pull out force when separated from a syringe as compared to conventional tip covers. The reduced pull out force may be achieved without altering the biocompatibility or other properties of the tip cover.

Further, the tip cover sealingly engages the syringe on which the cover is disposed. Such engagement prevents any contamination of the medicinal fluid disposed within the syringe from the outside environment, thereby assuring the container closure integrity. The tip cover further prevents any leakage of the medicinal fluid from the syringe. To that end, the tip cover having a reduce pull out force may not be unintentionally removed and must maintain the container closure integrity over time, such as after a long storage period.

To this end, the invention proposes a method for treating a tip cover for an injection device to reduce the pull out force of the tip cover. The method comprises applying a silicone oil to an inner surface of a tip cover. The inner surface of the tip cover is configured to engage the outer surface of a tip of an injection device. The method further comprises treating the silicone oil to form a cross-linked silicone coating on the inner surface of the tip cover.

Certain preferred but non-limiting features of the method for treating the tip cover described above are the following, taken individually or in combination:
the silicone oil comprises poly(dimethyl siloxane);
the tip cover comprises an elastomeric material, and the cross-linked silicone coating is disposed on the elastomeric material;
the elastomeric material of the tip cover comprises rubber;
the step of treating the silicone oil to form the cross-linked silicone coating comprises one of plasma treating, heat treating, or radiation treating the silicone oil;
the silicone oil has a viscosity in a range from 300 to 35,000 centiStokes (cSt), inclusive, or 25,000 to 35,000 cSt before the silicone oil is treated;
the step of applying a silicone oil to an inner surface of a tip cover comprises applying from 10 to 300 µg/cm² of the silicone oil to the inner surface of the tip cover, preferably from 20 to 200 µg/cm² of the silicone oil; and/or
a pull out force for removing the tip cover from the tip of the injection device is at least 5% less than and at most 35% less than a pull out force for removing a tip cover of a same configuration and having a same quantity of the silicone oil thereon that is not cross-linked.
The invention also proposes a tip cover comprising the cross-linked silicone coating formed by the aforementioned method.
Certain preferred but non-limiting features of the tip cover described above are the following, taken individually or in combination:
the tip cover comprises a needle shield having an inner needle shield formed of an elastomeric material and a rigid outer needle shield surrounding the inner needle shield;
the inner needle shield has an inner surface configured to sealingly engage a needle of an injection device therein;
the cross-linked silicone coating is disposed on the inner surface of the inner needle shield;
the tip cover comprises a unitary tip cap; and/or
the unitary tip cap comprises an inner surface configured to sealingly engage a luer tip of an injection device, and the cross-linked silicone coating is disposed on the inner surface of the unitary tip cap.

The invention also proposes an injection device comprising a barrel having a chamber configured to retain a medicinal fluid therein, which the barrel having a proximal end and a distal end, the distal end comprising a fluid passageway for passage of the medicinal fluid therethrough; and the aforementioned tip cover disposed on the distal end of the barrel.

### BRIEF DESCRIPTION OF THE DRAWINGS

The illustrations presented herein are not meant to be actual views of any particular component, device, or system, but are merely idealized representations which are employed to describe embodiments of the present invention. Other features, goals, and advantages of the present invention will appear more clearly upon reading of the detailed description that follows and with references to drawings provided by way of non-limiting examples wherein:
FIG. 1 is a perspective view of a tip cap;
FIG. 2 is a cross-sectional view of the tip cap of FIG. 1 mounted on an injection device;
FIG. 3 is a side view of an injection device having a tip cap thereon;
FIG. 4 is an isolated, cross-sectional view of a needle shield;
FIG. 5 is a perspective view of an injection device;
FIG. 6 is a side view of the injection device of FIG. 5 having a needle shield thereon;
FIG. 7 is a graph plotting pull out force as a function of time;
FIG. 8 is a graph plotting change in length of the injection device as a function of time; and
FIGS. 9 and 10 are graphs plotting force applied to a tip cap during assembly of the tip cap onto a syringe.

### DETAILED DESCRIPTION

As used herein, the term "proximal" refers to a location, such as a proximal end, that is nearer to a point of reference such as a point of contact of a user applying a force to a plunger rod of an injection device as described herein. As used herein, the term "distal" refers to a location, such as a distal end, that is farther from a point of reference such as a point of contact of the user applying a force to the plunger of the injection device as described herein. Thus, the terms "proximal" and "distal" refer to, for example, directions nearer to and farther from, respectively a user administering a medicinal fluid to a patient.

As used herein, the terms "axial," "axially," "longitudinal," and "longitudinally" generally mean and refer to a direction along or parallel to a longitudinal axis of an element(s) of the injection device described herein.

As used herein, the terms "radial," "radially," "lateral," and "laterally" generally mean and refer to a direction perpendicular to the central, longitudinal axis of the element(s) of the injection device described herein.

As used herein, the term "and/or" includes any and all combinations of one or more of the associated listed items.

As used herein, the term "configured" refers to a size, shape, material composition, material distribution, orientation, and arrangement of one or more of at least one structure and at least one apparatus facilitating operation of one or more of the structures and the apparatus in a pre-determined way.

As used herein, the term "tip cover" refers to a device that is disposable on a distal end of an injection device, such as a syringe, so as to cover this distal end. For instance, the tip cover may be a tip cap, a plastic rigid tip cap, a needle cover, etc. In the present disclosure, the term "tip cover" is used to refer separately or collectively to a tip cap as described in detail herein and to a needle cover as described in detail herein.

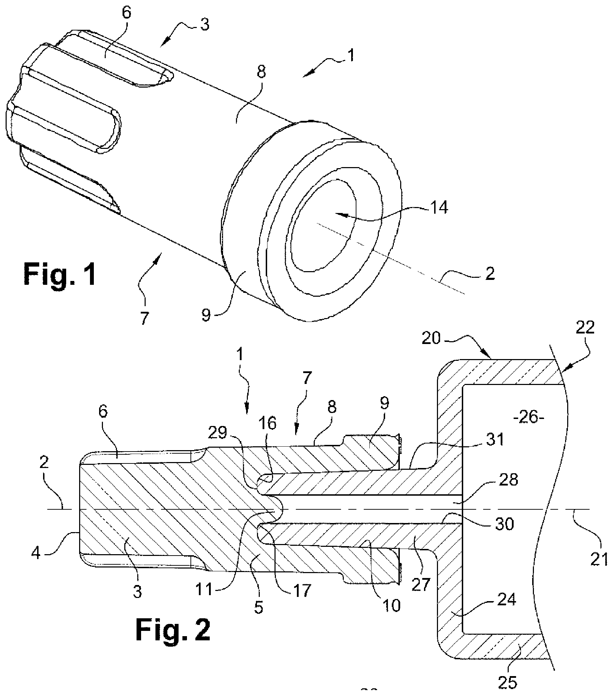

FIGS. 1-2 illustrates a tip cap 1 according to an embodiment of the present disclosure. In some embodiments, the tip cap 1 may be configured to be disposed on a luer tip of an injection device. The tip cap 1 may be known in the art as a unitary tip cap as the tip cap may be formed monolithically.

The tip cap 1 may comprise a cavity 14 defined by an inner surface 10 thereof. The inner surface 10 may be configured to engage an outer surface 31 of a tip 27 of an injection device 20 (FIG. 2). The cavity 14 may extend from a proximal end 9 of the tip cap 1 toward a distal end 4 along a longitudinal axis 2.

The tip cap 1 may comprise a closed distal portion 3 proximate the distal end 4 and an open proximal portion 7 proximate the proximal end 9. In some embodiments, the closed distal portion 3 may comprise a plurality of notches 6 circumferentially space apart along an exterior surface 8. The notches 6 create a ribbed exterior surface configured to be gripped by a user when the tip cap 1 is to be removed from an injection device 20. In other embodiments, the exterior surface 8 of the closed distal portion 3 may be smooth (i.e., lacking notches). The open proximal portion 7 may be configured as a cylindrical peripheral skirt such that the injection device 20 is receivable within the tip cap 1. The open proximal portion 7 may comprise the cavity 14.

The inner surface 10 of the tip cap 1 may be configured to seal a distal end 29 of the injection device 20. The inner surface 10 of the tip cap 1 may comprise a protrusion 11 that extends from a proximal end 5 of the closed distal end portion 3. The inner surface 10 of the tip cap 1 may further comprise an inner surface 16 of the closed distal portion 3. The inner surface 16 may extend annularly about the protrusion 11.

The tip cap 1 may be formed of an elastomeric material, which is a material having elastomeric properties. The tip cap 1 may be made of a thermoplastic elastomer (TPE) or rubber. Materials with elastomeric properties that are sterilizable are preferred.

Materials with elastomeric properties may be preferrable for sealing syringe tips because such materials may conform to the shape of the tip 27 of the injection device 20.

As illustrated in FIG. 2, the tip cap 1 may be disposed on and sealingly engaged with the injection device 20. In operation, the injection device 20 may be filled with a medicinal fluid. Prior to use, the tip cap 1 may be provided such that the medicinal fluid within a barrel 22 of the injection device 20 cannot flow out of the injection device 20. Accordingly, the tip cap 1 fluidly sealingly engages the injection device 20. Further, the tip cap 1 may prevent external contaminants from entering into the injection device 20 to maintain its container closure integrity.

The injection device 20 may comprise a barrel 22 extending axially along a longitudinal axis 21 between a distal end 24 and a proximal end 23. The barrel 22 is configured to retain a medicinal fluid within a cavity 26. The cavity 26 is defined by a cylindrical sidewall 25.

The distal end 24 may comprise a fluid passageway for passage of the medicinal fluid therethrough. More particularly, the injection device 20 may comprise a tip 27 having a fluid passageway 28 defined by an inner surface 30 of the tip 27.

The tip 27 of the injection device 20 may extend from the cylindrical sidewall 25 of the barrel 22. The tip 27 may comprise an annular, outer surface 31 and a distalmost surface 17. The fluid passage 28 may be in fluid communication with the chamber 26.

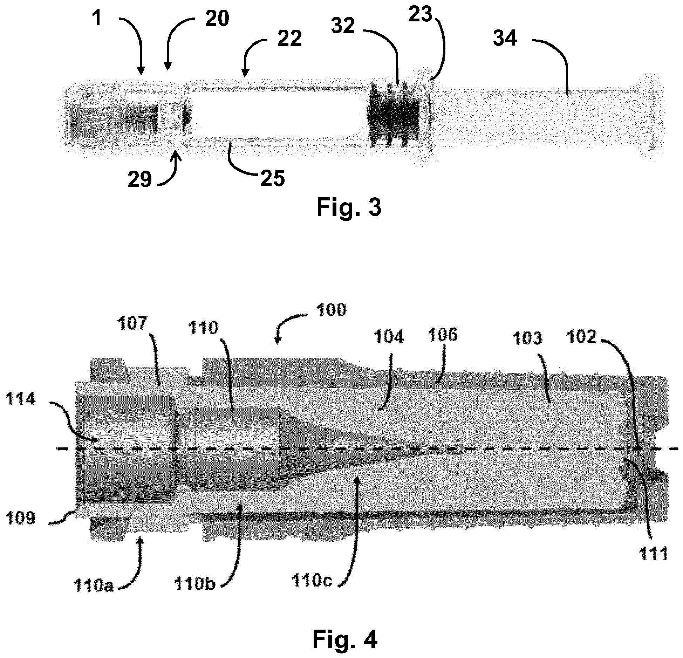

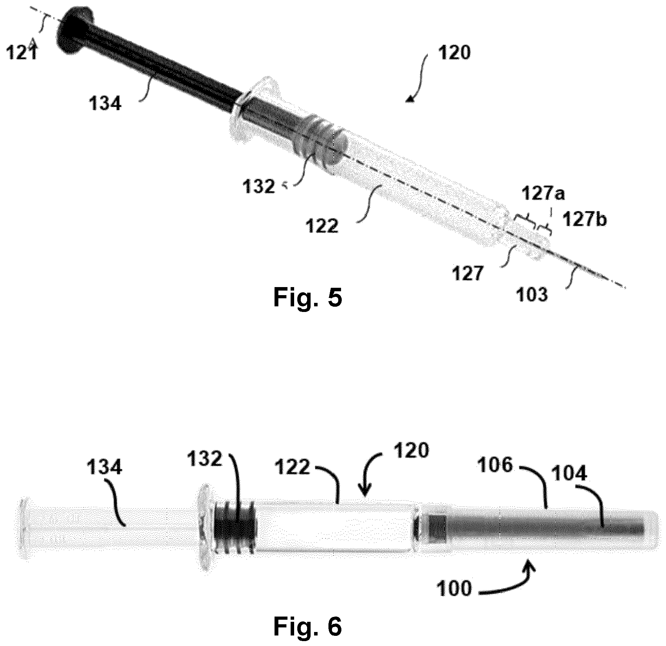

In operation, once the tip cap 1 is removed from the tip 27 of the injection device 20, a cannula (e.g., disposable needle) may be coupled to the tip 27 for injecting the medicinal fluid to a patient. The cannula may be coupled, such as by threading, to the distal end 24 via an adaptor, which may be snap-fitted or glued to the distal end 24 (see FIG. 3). Alternatively, the cannula may be directly stacked on the distal end 24 of the injection device 20 (see FIG. 5).

When the tip cap 1 is disposed on the tip 27 of the injection device 20, the inner surface 10 of the tip cap 1 may be engaged with the outer surface 31 of the tip 27. Further, the distalmost surface 17 of the tip 27 may abut against the inner surface 16 of the closed distal portion 3, and the protrusion 11 may be partially disposed within the fluid passage 28.

As illustrated in the side view of FIG. 3, the injection device 20 may comprise a stopper 32 disposed in the barrel 22 and coupled to a plunger rod 34. In operation, the plunger rod 34 and the stopper 32 may be axially displaced along the axis 21 within the barrel 22 to expel medicinal fluid from the chamber 26, through the fluid passage 28, into the needle, and to a patient. In FIG.3, the injection device 20 does not comprise a staked needle. Instead, an adaptor is mounted on the distal end of the barrel 22, enabling mounting of a needle on the injection device 20 prior to the injection. In that case when the injection device 20 comprises an adaptor, a plastic rigid tip cap may be mounted onto said adaptor in order to prevent external contaminants from entering into the injection device 20 to maintain its container closure integrity.

The plastic rigid tip cap may be formed of an elastomeric material, which is a material having elastomeric properties. The tip cap 1 may be made of a thermoplastic elastomer (TPE) or rubber. Materials with elastomeric properties that are sterilizable are preferred.

A material of the barrel 22 may vary depending on the medicinal fluid to be disposed therein. The barrel 22 may be formed of a polymeric material (e.g., plastic) or glass.

FIG. 4 illustrates a needle cover 100 according to an embodiment of the present disclosure. In some embodiments, the needle cover 100 may comprise an inner needle shield 104 and an outer needle shield 106 surrounding the inner needle shield 104.

The inner needle shield 104 may be formed of a flexible material. The outer needle shield 106 may be formed of a rigid material (e.g., a less flexible material than the inner needle shield 104).

The inner needle shield 104 may be formed of an elastomeric material, which is a material having elastomeric properties. The inner needle shield 104 may be made of a thermoplastic elastomer (TPE) or rubber. Materials with elastomeric properties that are sterilizable are preferred. The elastomeric material of the inner needle shield 104 may be pierceable by a needle 103 of the injection device 120.

The inner needle shield 104 comprises an inner surface configured to sealingly engage a needle of an injection device therein and to sealingly engage a tip of the injection device 120.

The outer needle shield 106 may be formed of a rigid plastic and may be configured to surround the inner needle shield 104.

The needle cover 100 may comprise a cavity 114 defined by an inner surface 110 thereof. Like the tip cap 1, the inner surface 110 may be configured to engage an outer surface of a tip 127 of the injection device 120 (FIG. 5).

In the embodiment of FIG.5, a needle 103 is coupled to the tip 127 of the barrel 122 before the needle cover 100 is mounted onto the injection device 120. Accordingly, the inner surface 110 of the needle cover 100 may be configured to engage the outer surface of the tip 127 of the injection device 120 and the needle 103 coupled to the tip 127 of the injection device 120.

The cavity 114 may extend from a proximal end 109 of the needle cover 100 toward a distal end 104 along a longitudinal axis 102.

The needle cover 100 may comprise a closed distal portion 103 and an open proximal portion 107. The open proximal portion 107 may be configured as a cylindrical peripheral skirt such that a portion of the injection device 120, typically the tip 127, is receivable within the needle cover 100. The open proximal portion 107 may comprise the cavity 114.

More particularly, the inner surface 110 of the needle cover 100 may be configured to fluidly seal a distal end of the injection device 120. The inner surface 110 of the needle cover 100 may vary in shape. By way of non-limiting example, the inner surface 110 may have a portion configured to engage with the tip 127 of the barrel 122 and another portion configured to engage with the needle 103.

As illustrated in FIG. 4, the inner surface 110 may comprise a first portion 110a, a second portion 110b, and a third portion 110c. The first portion 110a may be located proximate to the proximal end 109, the third portion 110c may be located proximal to the distal end 104, and the second portion 110b may be located between the first and third portions 110a, 110c.

The first portion 110a and second portions 110b may be configured to sealingly engage portions 127a, 127b, which have different dimensions, of the tip 127 of the barrel 122, and the third portion 110c may be configured to sealing engage the needle 103.

Alternatively, the needle cover 100 may have an inner needle shield configured to engage with a needleless syringe and having an outer needle shield formed of a rigid plastic and may be configured to surround the inner needle shield.

As illustrated in FIG. 6, the needle cover 100 may be disposed on and sealingly engaged with the injection device 120. In operation, the injection device 120 may be filled with a medicinal fluid. Prior to use, the needle cover 100 may be provided such that the medicinal fluid within the barrel 122 of the injection device 120 cannot flow out of the injection device 120.

The injection device 120 may comprise the barrel 122 extending axially along a longitudinal axis 121 between a distal end 124 and a proximal end 123. The barrel 122 is configured to retain a medicinal fluid within a chamber. Like the chamber 26, the chamber of the barrel 122 may be defined by a cylindrical sidewall and an annular wall.

The injection device 10 may comprise a stopper 132 disposed in the barrel 122 and coupled to a plunger rod 134. In operation, the plunger rod 134 and the stopper 132 may be axially displaced along the axis 121 within the barrel 122 to expel medicinal fluid from the chamber, through the fluid passage, into the needle 103, and to a patient.

According to the present disclosure, the tip cover may comprise a cross-linked silicone coating disposed on the inner surface 10, 110 thereof. The cross-linked silicone coating may be disposed on at least a portion of the inner surface 10, 110. For instance, a portion of the inner surface 10, 110 that engages an outer surface of the tip 27, 127 of the injection device 20, 120 may comprise the cross-linked silicone coating. Optionally, the entirety of the inner surface 10, 110 may comprise the cross-linked silicone coating.

To form the cross-linked silicone coating, a silicone oil is applied to at least a portion of the inner surface 10, 110. In some embodiments, more than one silicone oil may be applied to the inner surface 10, 110. For example, a first silicone oil having a first viscosity may be applied to the inner surface 10, 110, and a second silicone oil having optionally a second viscosity different from the first viscosity may be applied to the inner surface. One or more silicone oils may be applied by any suitable method including, but not limited to, spraying, dipping and washing in a water-silicone emulsion.

The silicone oil may comprise poly(dimethyl siloxane), or PDMS. In some embodiments, the silicone oil may consist of PDMS.

The silicone oil may have a viscosity in a range from 100 to 50,000 centistokes (cSt), inclusive, preferably from 300 to 35,000 cSt, inclusive, and, more particular, in a range from 1,000 to 30,000 cSt, inclusive. More specifically, the silicone oil may have a viscosity of 1,000 cSt or a viscosity of 30,000 cSt before the silicone oil is treated. As previously described, in some embodiments, a first silicone oil having a viscosity of 30,000 cSt and a second silicone oil of 1,000 cSt may be applied to the inner surface 10, 110. The foregoing viscosities are measured at 25°C with a standard viscosimeter.

A concentration of silicone oil applied to an inner surface of the tip cover may be in a range from 10 µg/cm² to 300 µg/cm² or, more particularly, in a range from 20 µg/cm² to 200 µg/cm². In some embodiments, the concentration of silicone oil may be about 25 µg/cm². In other embodiments, the concentration of silicone oil may be about 200 µg/cm². The concentration of silicone oil may be measured through extraction in a solvent followed by atomic emission spectroscopy.

After applying the silicone oil to the inner surface 10, 110, the silicone oil may be treated so as to cross-link the silicone oil into the silicone coating. Methods of treating the silicone oil to form the cross-linked silicone coating may comprise one of plasma treating, heat treating, or radiation treating the silicone oil.

In some embodiments, a plasma treatment is applied to the silicone oil in order to crosslink the silicone oil. The plasma treatment may be an oxidizing plasma treatment.

The plasma treatment may be carried out in an atmosphere comprising oxygen and argon. The plasma treatment is applied in order to crosslink the silicone oil into a lubricant coating. The plasma treatment is preferably applied in an oxidizing atmosphere.

According to a preferred embodiment, the atmosphere is a mixture of 15 to 30% of oxygen and 85 to 70% of argon, in term of partial pressures. More preferably, the atmosphere comprises 25% of oxygen and 75% of argon.

Generally speaking, the plasma may be produced by different techniques such as corona discharge, microwave, radio-frequency or any other convenient methods.

Preferably, a radio frequency plasma treatment is used, i.e. with a frequency ranging from 10 to 20 MHz, more preferably from 11 to 14 MHz. The power applied to generate the plasma may be comprised between 50 and 300 W, preferably between 100 and 250 W and the plasma treatment may be carried out at room temperature (i.e., 25°C) and under a vacuum ranging from 1 .33 to 13.3 Pa (10 to 100 mTorr) in absolute value.

The exposure time of the silicone oil layer to the plasma is typically between 10 to 40 seconds.

The above parameters depend on the plasma reactor geometry, a surface area and volume of the tip cover comprising the silicone coating, the arrangement of the devices inside the plasma reactor, the thickness of the silicone layer, a surface density of the silicone layer, etc.

As a consequence, tiny changes in parameters can lead to a different coating and the skilled person may select the parameters of the plasma treatment in order to optimize the treatment depending on the equipment used as well as on the devices to be treated.

As a result of the plasma treatment, the silicone oil is crosslinked to form a cross-linked coating on the inner surface 10, 110. Tip covers having a coating formed from cross-linking a quantity of silicone oil thereon may exhibit a pull out force for removing the tip cover from the tip of an injection device that is less than a pull out force for removing a tip cover of the same configuration and having the same quantity of silicone oil thereon that is not cross-linked. More particularly, the tip covers having the cross-linked silicone coating thereon may exhibit a pull out force that is at least 5% less than and at most 35% less than a pull out force for removing a tip cover of the same configuration and having the same quantity of silicone oil thereon that is not cross-linked. The foregoing reduced pull out forces are measured in a range of between 5 days and 9 weeks after the tip cover is disposed on the syringe. Such pull out forces may be measured before or after a sterilization process. More preferably, the tip covers having the cross-linked silicone coating thereon may exhibit a pull out force that is at least 10%, at least 15%, or at least 20% less than a pull out force for removing a tip cover of the same configuration and having the same quantity of silicone oil thereon that is not cross-linked. The pull out force is measured for each tip cover with a traction bench according to the procedure described below.

The pull out force required to remove the tip cover may vary based on the configuration of the tip cover. For example, a smaller pull out force may be required to remove a tip cap, as described with reference to FIGS. 1-3, from a syringe than the needle cover 100, as described with reference to FIGS. 4-6, from a syringe. With reference to the tip cap 10, the tip cap having the cross-linked silicone coating thereon may exhibit a pull out force in a range from 2 N to 10 N, inclusive.

### EXPERIMENTAL DATA

The following experimental data illustrates, by way of comparison, the effect of a cross-linked silicone coating according to the present disclosure on pull out force of a tip cap from a syringe. The pull out force is affected by an amount of silicone oil applied to an inner surface of a tip cap and/or as a function of cross-linking of the silicone oil that has been applied to the inner surface of the tip cap.

Experimental data is provided for the following samples:
- a tip cap lacking silicone oil ("No Silicone")
- a tip cap having approximately 25 µg/cm² of silicone oil having a viscosity of 30,000 cSt measured at 25°C (also known in the art as "transport silicone") disposed on an inner surface thereof as described herein ("Reference")
- a tip cap having approximately 25 µg/cm² of silicone oil having a viscosity of 30,000 cSt measured at 25°C disposed on an inner surface thereof and which silicone oil has been crosslinked according to a plasma treatment as described herein ("Ref+Cross-linked")
- a tip cap having approximately 25 µg/cm² of silicone oil having a viscosity of 30,000 cSt measured at 25°C and 175 µg/cm² of silicone oil having a viscosity of 1,000 cSt measured at 25°C disposed on an inner surface thereof as described herein ("Sil2")
- a tip cap having approximately 25 µg/cm² of silicone oil having a viscosity of 30,000 cSt measured at 25°C and 175 µg/cm² of silicone oil having a viscosity of 1,000 cSt measured at 25°C disposed on an inner surface thereof and which silicone oils have been crosslinked according to a plasma treatment as described herein ("Sil2+Cross-linked")

The tip cap for each of the foregoing samples was the same including the following characteristics:
- Material: styrene-butadiene rubber
- Configuration: as described herein with reference to the tip cap of FIGS. 1-3

To form the cross-linked silicone coating of samples Reference and Ref+Cross-linked, approximately 25 µg/cm² of silicone oil having a viscosity of 30,000 cSt is applied to the inner surface of the tip cap. The silicone oil of the Reference samples remains in a non-cross-linked state while the silicone oil of the Ref+Cross-linked sample is subject to the plasma treatment process having the following preferred conditions:
- plasma type: capacitively coupled plasma driven by a radiofrequency power supply at 13.56 MHz;
- treatment time: 30 s;
- power: 200 W;
- pressure: 8 Pa (0.06 Torr);
- atmosphere: 15/75 partial pressure of O₂ and Ar, respectively.

To form the cross-linked silicone coating of samples Sil2 and Sil2+Cross-linked, approximately 25 µg/cm² of silicone oil having a viscosity of 30,000 cSt is applied to the inner surface of the tip cap and an additional 175 µg/cm² of silicone oil having a viscosity of 1,000 cSt is subsequently applied to the inner surface of the tip cap. The silicone oil of the Sil2 samples remain in a non-cross-linked state while the silicone oil of the Sil2+Cross-linked sample is subject to the plasma treatment process of the foregoing conditions.

### Pull out force

Measurements of the force needed to remove the tip cap from a glass syringe were carried out. Table 1 sets forth for tip cap of the sample types set forth above the number of tip cap samples on which measurements were taken and the time delay between assembly of the tip caps on the syringe and the measurements on each sample. For the samples which have been steam sterilized, the sterilization process was undertaken one week after the assembly of the tip cap on the syringe, and the POF measurements have been undertaken one week after the sterilization process.

**Table 1. Sample Sizes**

| **Sample** | **Time** | **Number** |
|---|---|---|
| No Silicone | 3 weeks | 18 |
| | 9 weeks | 19 |
| Reference | 5 days | 17 |
| | 3 weeks | 40 |
| | 9 weeks | 40 |
| | Steam Sterilization | 14 |
| Ref+Cross-linked silicone | 5 days | 20 |
| | 3 weeks | 37 |
| | 9 weeks | 39 |
| | Steam Sterilization | 20 |
| Sil2 | 5 days | 13 |
| | 3 weeks | 22 |
| | 9 weeks | 29 |
| | Steam Sterilization | 13 |
| Sil2+ Cross-linked silicone | 5 days | 14 |
| | 3 weeks | 19 |
| | 9 weeks | 28 |
| | Steam Sterilization | 24 |

The pull out force test was performed with a traction bench. The method comprises the steps of:
- placing the syringe on a holder,
- holding the tip cap with pneumatic jaws, and
- subsequently, pulling the syringe at a constant displacement rate to remove the tip cap.

The force needed to remove the tip cap is recorded, as a function of the displacement of the syringe. Measurements of the force needed to remove the tip cap from the syringe were carried out according to ISO standard 11040-4:2015 Annex G.6 with two exceptions: 1) the tip was oriented in a downward direction rather than an upward direction as set forth in the aforementioned ISO standard and 2) the grip length was greater than 2 mm while the ISO standard utilizes a 2 mm grip length. The displacement rate was 500 mm/min.

The pull out force is the maximum force recorded during the pull out force test described.

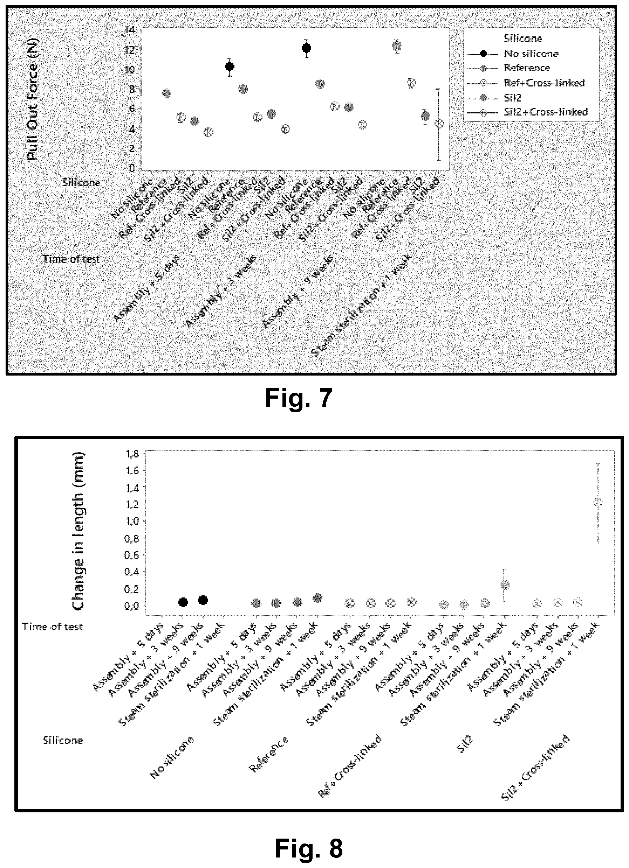

FIG. 7 plots the pull out force of a tip cap measured after a certain period of time had passed since the tip cap was disposed on the syringe and after steam sterilization. The pull out force (POF) values in Newtons (N) required to remove the tip cap were recorded at four times:
a) 5 days after the tip cap is disposed on the syringe, b) 3 weeks after the tip cap is disposed on the syringe, c) 9 weeks after the tip cap is disposed on the syringe, and d) after a steam sterilization process. The steam sterilization process may take place at a temperature of between 120°C and 125°C for 30-40 minutes.

Table 2 below provides pull out force values measured for the foregoing samples as illustrated in FIG. 7.

**Table 2. Pull Out Force Measurements**

| **Time after assembly** | **Sample** | **Mean (N)** | **Standard Deviation (N)** | **Minimum (N)** | **Maximum (N)** |
|---|---|---|---|---|---|
| **5 days** | Reference | 7.4 | 0.58 | 6.8 | 9.0 |
| | Ref+Cross-linked | 5.0 | 1.01 | 3.9 | 7.4 |
| | Sil2 | 4.6 | 0.58 | 3.8 | 5.6 |
| | Sil2+Cross-linked | 3.5 | 0.61 | 2.4 | 4.8 |
| **3 weeks** | No Sil | 10.3 | 1.80 | 7.9 | 13.4 |
| | Reference | 7.9 | 0.80 | 5.9 | 9.6 |
| | Ref+Cross-linked | 5.1 | 0.79 | 3.8 | 6.5 |
| | Sil2 | 5.4 | 0.71 | 3.8 | 6.5 |
| | Sil2+Cross-linked | 3.8 | 0.46 | 2.8 | 4.7 |
| **9 weeks** | No Sil | 12.2 | 1.95 | 8.7 | 16.7 |
| | Reference | 8.5 | 0.81 | 6.6 | 10.5 |
| | Ref+Cross-Linked | 6.2 | 0.98 | 4.4 | 8.2 |
| | Sil2 | 6.1 | 0.65 | 3.4 | 6.8 |
| | Sil2+Cross-Linked | 4.3 | 0.59 | 2.4 | 5.2 |
| **1 week after Steam Sterilization** | Reference | 12.4 | 1.26 | 9.9 | 14.4 |
| | Ref+Cross-linked | 8.6 | 1.00 | 6.9 | 10.3 |
| | Sil2 | 5.1 | 1.29 | 3.6 | 8.1 |
| | Sil2+Cross-linked | 4.4 | 2.29 | 1.0 | 6.1 |

These results illustrate that a cross-linked silicone coating on the inner surface of the tip cap results in a decrease in pull out force. The pull out force decreases as result of silicone oil being cross-linked when samples having the same quantity of silicone oil disposed thereon are compared (e.g., comparing Reference to Ref +Cross-linked and Sil2 to Sil2+Cross-linked). On average, tip caps having the cross-linked silicone coating have a pull out force that is approximately 25% lower compared to tip caps having silicone oil that is not cross-linked. The tip cap having the cross-linked silicone coating may have a pull out force that is at least approximately 14% less and at most approximately 35% less than a pull out force for removing a tip cover of the same configuration and having the same quantity of silicone oil thereon that is not cross-linked a pull out force. As can also be seen from FIG. 7 and Table 2, as compared to tip caps lacking any silicone oil, the presence of the cross-linked silicone coating decreases the pull out force by about 50% or more.

Tip caps according to the present disclosure exhibit a pull out force for removing the tip cover from the tip of an injection device in a range from 2 N to 10 N.

As seen from FIG. 7 and Table 2, steam sterilization results in an increase in pull out force. Nonetheless, for similar quantities of silicone, tip caps having a cross-linked silicone coating thereon have a lower pull out force relative to tip caps having a non-cross-linked silicone oil thereon even after steam sterilization.

In the normal course of preparing injection devices for use, the standard in the pharmaceutical industry is to sterilize the injection devices, for instance by steam-sterilization or by ethylene-oxide sterilization. Typically, the tip cap is applied to the end of the syringe prior to such steam-sterilization or ethylene-oxide sterilization. While decreasing pull out force is desirable such that the tip cap is easily removed when a pulling force is applied thereto, the extent to which the pull out force can be reduced is limited by undesirable removable of the tip cap when a pulling force is not applied. For example, a tip cap that has been configured in a manner that reduces the pull out force may be displaced relative to the syringe on which it is disposed during a steam-sterilization process or later such as during storage of the syringe. Such displacement is undesirable as displacement of the cap hinders the container closure integrity.

FIG. 8 is a plot of the change in length of the injection device over the same times indicated with reference to FIG. 7. The measured change in length of the injection device is attributable to movement of the tip cap relative to the syringe during storage. Table 3 provides average values of the change in length as plotted in FIG. 8.

**Table 3. Tip Cap Displacements**

| **Time** | **Family** | **Mean (mm)** |
|---|---|---|
| **5 days** | Reference | 0.01 |
| | Ref+Cross-linked | 0.01 |
| | Sil2 | 0 |
| | Sil2+Cross-linked | 0.01 |
| **3 weeks** | No Sil | 0.02 |
| | Reference | 0.01 |
| | Ref+Cross-linked | 0.01 |
| | Sil2 | 0 |
| | Sil2+Cross-linked | 0.02 |
| **9 weeks** | No Sil | 0.05 |
| | Reference | 0.03 |
| | Ref+Cross-linked | 0.02 |
| | Sil2 | 0.02 |
| | Sil2+Cross-linked | 0.03 |
| **Steam sterilization** | Reference | 0.08 |
| | Ref+Cross-linked | 0.03 |
| | Sil2 | 0.24 |
| | Sil2+Cross-linked | 1.21 |

As illustrated in FIG. 8 and listed in Table 3, the tip caps of each of the samples exhibit acceptably minimal displacement after 5 days, 3 weeks, and 9 weeks of storage. After steam sterilization, the displacement of the tip caps is limited and within an acceptable range for the Reference and Ref+Cross-linked samples. However, the samples having additional non-cross-linked and cross-linked silicone (i.e., Sil2 and Sil2+Cross-linked) exhibit a larger tip cap displacement after steam sterilization. A steam sterilization process contributes to movement of the tip cap relative to the syringe, and the samples having additional non-crosslinked and cross-linked silicone (i.e., Sil2 and Sil2+Cross-linked) exhibit a lower pull out force and are, thus, more prone to tip cap displacement. The results show a larger tip cap movement that is sufficiently sufficient to potentially jeopardize the container closure integrity. Thus, in a reduced movement of the tip cap relative to the syringe while also lowering the pull out force, cross-linking a minimal amount of silicone may be preferrable to adding additional silicone.

### Stick-slip during assembly

As previously discussed, tip caps must be disposed on the syringe. When the tip cap is disposed on the syringe, a stick-slip phenomenon may be observed. Stick-slip phenomenon may be observed when two objects slide relative to each other such as movement of the tip cap relative to the syringe during assembly.

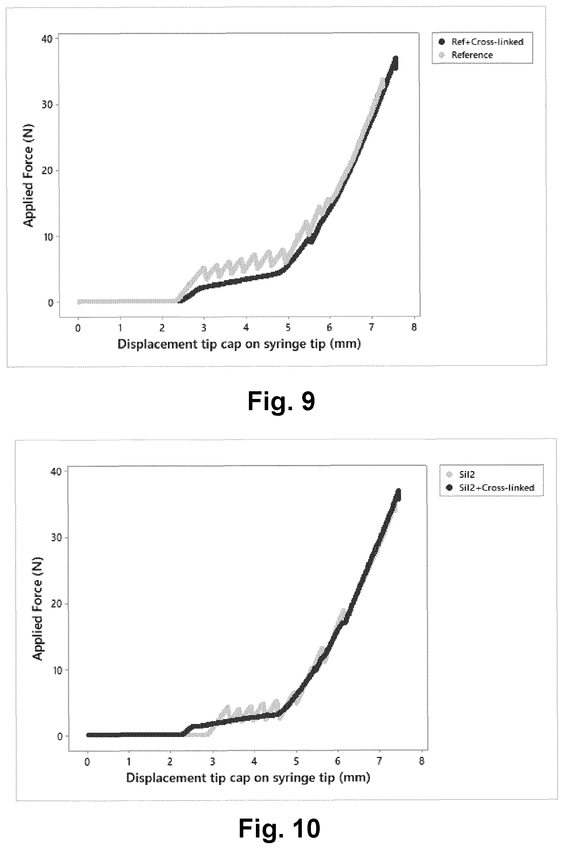

This stick-slip phenomenon impacts the automation and processability of tip cap assembly with the syringe. FIGS. 9 and 10 are plots of the force applied to a tip cap for Reference samples and Ref+Cross-linked samples, respectively, during assembly on the syringe as a function of distance traveled by the tip cap. FIGS. 9 and 10 illustrates that a cross-linked silicone coating limits or even eliminates the stick-slip phenomenon of the tip caps with the syringe relative to non-cross-linked silicone oil. The most relevant portion of the curves is the portion displaying the force applied between around 2 mm and 5 mm. At distances below 2 mm, the tip cap is not yet mounted on the tip of the syringe. At distances above 5 mm, the tip cap abuts (e.g., presses against) the tip of the syringe and is compressed. The stick-slip phenomenon is visible on the plot for samples which do not include cross-linked silicone (e.g., Reference). In the plot, the force required to continue moving the tip cap rapidly increases and decreases (e.g., fluctuates) between 2 mm and 5 mm for the reference sample which lacks cross-linked silicone. This behavior is not exhibited over the same distance for samples having cross-linked silicone thereon. Thus, providing a cross-linked silicone coating on the inner surface of the tip caps improves the processability of the tip caps with the syringe.

While the present disclosure has been described herein with respect to certain illustrated embodiments, those of ordinary skill in the art will recognize and appreciate that it is not so limited. Rather, many additions, deletions, and modifications to the illustrated embodiments may be made without departing from the scope of the disclosure, including legal equivalents thereof. In addition, features from one embodiment may be combined with features of another embodiment while still being encompassed within the scope of the invention as contemplated by the inventors.

## Claims

1. A method for treating a tip cover (1, 100) for an injection device (20, 120) to reduce the pull out force of the tip cover (1, 100), comprising:
applying a quantity of silicone oil to an inner surface (10, 110) of a tip cover (1, 100), the inner surface (10, 110) configured to engage an outer surface of a tip of an injection device; and
treating the silicone oil to form a cross-linked silicone coating on the inner surface (10, 110) of the tip cover (10).

2. The method of claim 1, wherein the silicone oil comprises poly(dimethyl siloxane).

3. The method of claim 1 or 2, wherein the tip cover comprises an elastomeric material, the cross-linked silicone coating disposed on the elastomeric material.

4. The method of claim 3, wherein the elastomeric material comprises rubber.

5. The method of any of claims 1-4, wherein treating the silicone oil to form the cross-linked silicone coating comprises one of plasma treating, heat treating, or radiation treating the silicone oil.

6. The method of any of claims 1-5, wherein applying a quantity of silicone oil to an inner surface (10, 110) of a tip cover comprises applying a silicone oil having a viscosity in a range from 300 to 35,000 centiStokes (cSt), inclusive, or, preferably, in a range from 25,000 to 35,000 cSt, inclusive.

7. The method of any of claims 1-6, wherein applying a quantity of silicone oil to an inner surface (10, 110) of a tip cover comprises applying from 10 to 300 µg/cm² of the silicone oil to the inner surface (10, 110) of the tip cover, preferably from 20 to 200 µg/cm² of the silicone oil.

8. The method of any of claims 1-7, wherein a pull out force for removing the tip cover from the tip of the injection device is in a range from 5% to 35% less than a pull out force for removing a tip cover of a same configuration and having a same quantity of the silicone oil thereon that is not cross-linked.

9. A tip cover comprising the cross-linked silicone coating formed by the method of any of claims 1-8.

10. The tip cover (10, 110) of claim 9, comprising a needle shield (110) having:
an inner needle shield formed of an elastomeric material, the inner needle shield having an inner surface configured to sealingly engage a needle of an injection device therein; and
a rigid outer needle shield surrounding the inner needle shield;
wherein the cross-linked silicone coating is disposed on the inner surface of the inner needle shield.

11. The tip cover of claim 9, comprising a unitary tip cap, the unitary tip cap comprising an inner surface configured to sealingly engage a luer tip of an injection device, wherein the cross-linked silicone coating is disposed on the inner surface of the unitary tip cap.

12. An injection device comprising:
a barrel having a cavity configured to retain a medicinal fluid therein, the barrel having a proximal end and a distal end, the distal end comprising a fluid passageway for passage of the medicinal fluid therethrough; and
the tip cover of any of claims 9-11 disposed on the distal end of the barrel.
